# EUROPÄISCHE PATENTANMELDUNG

(11) **EP 1 747 817 A1**
(43) Veröffentlichungstag der Anmeldung: **31.01.2007**
(21) Anmeldenummer: 05016273.4
(22) Anmeldetag: 27.07.2005
(51) Int. Cl.: B06B 1/02, A61H 23/02

(54) **System und Verfahren zur Erzeugung von Ultraschallwellen mit zumindest zwei unterschiedlichen Frequenzen**

(71) Anmelder: Wellcomet GmbH, 75180 Pforzheim (DE)
(72) Erfinder: Die Erfindernennung liegt noch nicht vor
(74) Vertreter: Brommer, Hans Joachim

(57) **Zusammenfassung**

Die Erfindung betrifft ein System und ein Verfahren zur Erzeugung von Ultraschallwellen, insbesondere zur kosmetischen und therapeutischen Behandlung mit einem elektrisch angesteuerten Ultraschallkopf, der Ultraschallwellen mit zumindest zwei unterschiedlichen Frequenzen erzeugt. Wesentlich ist, dass mittels einer Steuereinheit die erzeugten Ultraschallwellen permanent oder zumindest während vorgegebener Zeitspannen zwischen den zumindest zwei vorgegebenen Frequenzwerten umgeschaltet werden.

## Beschreibung

Die Erfindung betrifft ein System zur Erzeugung von Ultraschallwellen gemäß dem Oberbegriff des Anspruchs 1, sowie ein Verfahren zur Erzeugung von Ultraschallwellen gemäß dem Oberbegriff des Anspruchs 13.

Ultraschallwellen werden zur Beaufschlagung von biologischem Gewebe für medizinische, therapeutische oder kosmetische Anwendungen, sowie für Forschungszwecke benutzt. Üblicherweise werden mit dem Begriff Ultraschallwellen Schwingungen mit einer Frequenz größer 20 kHz bezeichnet. Häufig ist hierbei die Verwendung von niederfrequenten Ultraschallfrequenzen im Bereich von 20 kHz bis 100 kHz und die Verwendung von hochfrequenten Ultraschallfrequenzen über 0,8 MHz.

Systeme zur Erzeugung von Ultraschallwellen für die Behandlung von biologischem Gewebe weisen üblicherweise einen Signalgenerator auf, welcher zur Erzeugung von periodischen elektrischen Signalen dient. Ferner umfassen typische Systeme einen Ultraschallkopf, welcher mittels einer elektrischen Leitung mit einem Signalausgang des Signalgenerators verbunden ist. Der Ultraschallkopf besitzt einen Bereich zur Auflage auf das biologische Gewebe, wobei im Ultraschallkopf in der Nähe dieses Auflagebereiches die vom Signalgenerator erzeugten periodischen elektrischen Signale in Ultraschallwellen umgewandelt werden.

Typischerweise ist ein Ultraschallkopf auf die Umwandlung von elektrischen Signalen mit einer festen Frequenz ausgelegt. Es sind jedoch auch Ultraschallköpfe bekannt, welche elektrische Signale mit unterschiedlichen Frequenzen in entsprechende Ultraschallwellen umwandeln können:

Die US 5,460,595 beschreibt ein System zur Erzeugung von Ultraschallwellen, bei dem der Signalgenerator und der Ultraschallkopf derart ausgelegt sind, dass Ultraschallwellen mit drei verschiedenen Frequenzen erzeugt werden können, ohne dass der Ultraschallkopf ausgetauscht werden müsste.

Dieses System stellt dem Anwender drei verschiedene Ultraschallfrequenzen zur Verfügung, um drei verschiedene Eindringtiefen in das biologische Gewebe zu bewirken.

Wie tief und mit welcher Intensität der Ultraschall in den Körper eindringt, hängt von der Frequenz der Ultraschallwelle ab. Ein Maß für die Eindringtiefe ist die so genannte Halbwertstiefe, das heißt die Strecke, nach der sich die Schallintensität in dem biologischen Gewebe auf 50 % durch Absorption vermindert hat. Je geringer die Frequenz, desto höher die Eindringtiefe. Für typisches biologisches Gewebe eines Menschen beträgt die Halbwertstiefe einer Ultraschallwelle mit Frequenz 3 MHz etwa 1 cm und für eine Ultraschallwelle mit der Frequenz 1 MHz etwa 3 cm.

Die Halbwertstiefe ist unabhängig von der eingestrahlten Intensität, wohingegen die bis zu einer gewissen Tiefe in dem biologischen Gewebe absorbierte Energie sowohl von der Frequenz als auch von der Intensität der Ultraschallstrahlung abhängt.

Mit dem bekannten System lässt sich somit die Halbwertstiefe mittels der benutzten Ultraschallfrequenz und die bis zu einer bestimmten Tiefe absorbierte Energie mittels der gewählten Intensität variieren. Wünschenswert wäre es jedoch, bei der Ultraschallbehandlung die Massagewirkung, das heißt die Relativbewegung einzelner Teile des behandelten biologischen Gewebes zueinander beeinflussen zu können.

Der Erfindung liegt daher die Aufgabe zugrunde, ein System und ein Verfahren zur Erzeugung von Ultraschallwellen zu schaffen, welches die Möglichkeiten der Beeinflussung von biologischem Gewebe durch Ultraschallwellen erweitert, insbesondere neue Massagewirkungen ermöglicht.

Diese Aufgabenstellung wird durch ein System zur Erzeugung von Ultraschallwellen gemäß Anspruch 1 und ein Verfahren zur Erzeugung von Ultraschallwellen gemäß Anspruch 13 gelöst. In den Unteransprüchen 2 bis 12 finden sich vorteilhafte Ausgestaltungen des erfindungsgemäßen Systems und in den Unteransprüchen 14 bis 16 finden sich vorteilhafte Ausführungen des erfindungsgemäßen Verfahrens.

Der Kern der vorliegenden Erfindung besteht also darin, nicht mehr mit einer oder mehreren gleich bleibenden Frequenzen zu arbeiten, sondern ständig oder zumindest während vorgegebener Zeitspannen zwischen den Frequenzen hin und her zu wechseln.

Beträgt die Anzahl der von dem Signalgenerator erzeugten Frequenzen 2, so wird zwischen der ersten und der zweiten Frequenz hin- und hergewechselt. Sofern die Anzahl der erzeugten Frequenzen größer 2 ist (*f*₁, ..., fₙ), so wird durch eine Reihenfolge die Abfolge der Frequenzen festgelegt, also beispielsweise das *f*₂ auf *f*₁ folgt, f₃ auf f₂, ... und f₁ auf fₙ. Selbstverständlich sind auch andere Abfolgen möglich. Wesentlich ist, dass bei einem Komplettdurchlauf der Reihenfolge für jede Frequenz mindestens für eine vorgegebene Zeitspanne Ultraschallwellen erzeugt werden.

Im Gegensatz zum Stand der Technik wird bei dem erfindungsgemäßen System zur Erzeugung von Ultraschallwellen somit nicht eine Ultraschallwelle mit permanent gleichbleibender Frequenz erzeugt, sondern es wird zwischen Ultraschallwellen mit mindestens zwei Frequenzen gewechselt. Dies ermöglicht neuartige Massagewirkungen, wie folgende Überlegung zeigt:

Durch Ultraschallwellen entstehen in dem bestrahlten biologischen Gewebe abwechselnd Überdruck und Unterdruck. Die Höhe dieses Druckes hängt wesentlich von der Intensität der Ultraschallstrahlung und von der Dichte des bestrahlten biologischen Gewebes ab.

Die Höhe des Drucks ist jedoch nicht frequenzabhängig. Die Ultraschallbeaufschlagung führt also in dem biologischen Gewebe zu Bereichen mit Überdruck und Bereichen mit Unterdruck, wobei der Druckwert, das heißt insbesondere die Differenz des Drucks zwischen den Bereichen mit Überdruck und den Bereichen mit Unterdruck frequenzunabhängig ist.

Frequenzabhängig ist jedoch die Entfernung der Bereiche mit Überdruck und der Bereiche mit Unterdruck. Zwei Bereiche mit Überdruck sind in etwa um eine Strecke voneinander entfernt, welche der Wellenlänge der verwendeten Ultraschallstrahlung entspricht. Etwa mittig zwischen diesen Bereichen mit Überdruck, das heißt in etwa eine halbe Wellenlänge entfernt, befindet sich ein Bereich mit Unterdruck. Je höher die Frequenz, desto geringer die Wellenlänge und damit der Abstand zwischen Bereichen mit Über- und Unterdruck. Auch wenn sich die Höhe des Drucks mit der Frequenz der verwendeten Ultraschallstrahlung nicht ändert, so ändert sich jedoch der Abstand zwischen Bereichen mit Über- und Unterdruck und damit der Druckgradient im Gewebe.

Bei herkömmlichen Systemen zur Erzeugung von Ultraschallstrahlung wird mit einer konstanten Frequenz gearbeitet, das heißt der Druckgradient im Gewebe ändert sich nicht. Bei der erfindungsgemäßen Vorrichtung wird jedoch zwischen mindestens zwei Frequenzen gewechselt, so dass aufgrund der unterschiedlichen Wellenlängen die Bereiche mit Über- und Unterdruck mit einem Frequenzwechsel an unterschiedlichen Orten auftreten und somit auch eine Variation des Druckgradienten stattfindet.

Wenn beispielsweise die Frequenz des Ultraschalls bei gleichbleibender Intensität von 1 MHz auf 3 MHz verändert wird, so bleiben die absoluten Druckwerte im bestrahlten Gewebe gleich, die Größe einer Mikromassageeinheit (d. h. der Abstand zwischen einem Bereich mit Überdruck und einem Bereich mit Unterdruck, welcher der halben Wellenlänge der gewählten Frequenz entspricht) ändert sich durch den Frequenzwechsel jedoch von 0,75 mm auf 0,25 mm. Durch den Frequenzwechsel ist somit eine dynamische Modellierung der Massagewirkung bei Ultraschallbehandlung möglich.

Die Geschwindigkeit, mit der zwischen den mindestens zwei Frequenzen des Signalgenerators gewechselt wird, kann durch eine Umschaltfrequenz angegeben werden. Die Umschaltfrequenz gibt an, wie oft pro Sekunde ein Frequenzwechsel stattfindet, d. h. von der aktuellen Ultraschallfrequenz zu der nächsten Umschaltfrequenz in der oben beschriebenen Reihenfolge für den Wechsel übergegangen wird.

Die Umschaltfrequenz sollte hoch genug gewählt werden, so dass auch bei einem Bewegen des Ultraschall-Kopfes während der Behandlung über das biologische Gewebe hinweg an jeder Stelle eine Behandlung mit den mindestens zwei Ultraschallfrequenzen stattfindet. Die Umschaltfrequenz sollte daher mindestens 10 Hz betragen.

In einer bevorzugten Ausführungsform ist die Steuereinheit derart ausgeführt, dass die Umschaltfrequenz im Bereich von 10 Hz bis 10 kHz liegt. Als besonders vorteilhaft hat sich der Bereich von 100 Hz bis 2000 Hz erwiesen.

Grundsätzlich stehen dem Fachmann für die Erzeugung der Ultraschallwellen wie auch für die Umschaltung zwischen den unterschiedlichen Frequenzen verschiedene Möglichkeiten zur Verfügung. Besonders zweckmäßig ist es, wenn mit einem Signalgenerator gearbeitet wird, der zumindest zwei Frequenzen erzeugt, die in Ultraschallwellen mit entsprechenden Frequenzen umgewandelt werden, und dass die Steuereinheit die Umschaltung zwischen den Frequenzen bewirkt. In erster Linie ist hierbei an eine Umschaltung zwischen den Frequenzen der elektrischen Signale des Signalgenerators gedacht. Es liegt aber auch im Rahmen der Erfindung, stattdessen oder zusätzlich zwischen den Frequenzen der Ultraschallwellen umzuschalten.

Bei der erstgenannten Alternative kann der Signalgenerator einen Oszillator und eine Frequenz-Modulationseinheit aufweisen, die aus der Oszillatorfrequenz zumindest zwei verschiedene Frequenzen erzeugt. In diesem Fall braucht der Oszillator nur eine Frequenz erzeugen, aus der die Modulationseinheit dann die gewünschten unterschiedlichen Frequenzen ableitet.

In einer vorzugsweisen Ausführungsform weist der Signalgenerator einen Oszillator, einen Frequenzcontroller und eine Verstärkungseinheit auf. Der Oszillator erzeugt die Grundschwingung, wobei die Frequenz, mit der der Oszillator schwingt, über den mit dem Oszillator verbundenen Frequenzcontroller reguliert wird. Der Ausgang des Oszillators ist mit dem Eingang der Verstärkungseinheit verbunden, so dass am Ausgang der Verstärkungseinheit das Schwingungssignal des Oszillators in verstärkter Form vorliegt. Der Verstärkungsfaktor kann an der Verstärkungseinheit vorgegeben werden. Der Ausgang der Verstärkungseinheit ist mit dem Signalausgang des Signalgenerators verbunden, sodass das verstärkte Oszillatorsignal an den Ultraschallkopf weitergeleitet wird.

In einer weiteren vorzugsweisen Ausführungsform ist die Steuereinheit als Mikrocontroller ausgeführt, das heißt als Kontrolleinheit, welche eine elektronische Steuerung umfasst. Der Mikrocontroller kann beispielsweise durch einen Computer realisiert sein. Über einen Steuerausgang ist der Mikrocontroller mit dem Eingang des Frequenzcontrollers verbunden, sodass der Mikrocontroller letztendlich die Frequenz vorgibt, welche am Signalausgang des Signalgenerators anliegt.

Weiterhin ist es vorteilhaft, wenn die Steuereinheit zusätzlich die Intensität der Ultraschallwelle steuert. Hierzu kann die Steuereinheit zum einen mit einem Steuereingang der Verstärkungseinheit verbunden sein und zum anderen über einen Signaleingang mit einem Signalausgang der Verstärkungseinheit verbunden sein. Über den Signaleingang der Steuereinheit kann somit die tatsächliche Intensität des am Signalausgang des Signalgenerators anliegenden Signals überprüft werden und entsprechend kann der Verstärkungsfaktor korrigiert und entsprechend an den Steuereingang der Verstärkungseinheit weitergeleitet werden, sodass am Signalausgang ein Signal mit einer vorgegebenen Intensität anliegt.

Darüber hinaus ist es vorteilhaft, wenn das System zur Erzeugung von Ultraschallwellen mindestens zwei Ultraschallköpfe mit unterschiedlicher Größe aufweist. Ultraschallköpfe mit unterschiedlicher Größe haben eine unterschiedlich große Auflagefläche zur Auflage auf das zu bestrahlende biologische Gewebe. Je nach Form des Gewebes und abhängig von der gewünschten zu bestrahlenden Fläche kann so ein passender Ultraschallkopf ausgewählt werden. Hierzu ist der Signalausgang des Signalgenerators derart ausgeführt, dass wahlweise ein oder die mindestens zwei Ultraschallköpfe gleichzeitig mit dem Signalausgang verbunden werden können. Dadurch kann entweder ein passender Ultraschallkopf zur Verwendung ausgewählt werden oder es können mehrere Ultraschallköpfe angeschlossen werden, sodass gleichzeitig mehrere Stellen des biologischen Gewebes mit Ultraschallstrahlung beaufschlagt werden können.

In einer weiteren vorteilhaften Ausführungsform weist das System mindestens zwei Ultraschallköpfe auf, welche mit dem Signalausgang des Signalgenerators verbunden werden können, wobei der Signalausgang eine Signalsteuereinheit umfasst, welche automatisch das Signal an jeweils einen der angeschlossenen Ultraschallköpfe weiterleitet. Die Signalsteuereinheit weist einen Speicher zum Abspeichern von Behandlungsprogrammen auf, in denen die Behandlungsdauer und eventuell weitere Parameter wie Stärke und Frequenz der Ultraschallstrahlung oder Umschaltfrequenz für einen bestimmten Ultraschallkopf vorgegeben sind. Die Signalsteuereinheit ist hierfür mit einer Steuereinheit der Ultraschallköpfe verbunden, zur Steuerung der genannten weiteren Parameter. Nach Ablauf der vorgegebenen Behandlungsdauer schaltet die Signalsteuerung automatisch auf den nächsten im Programm angegebenen Ultraschallkopf um und gibt dies durch ein optisches und/oder akustisches Signal an. Weiterhin übermittelt die Signalsteuerung die weiteren Parameter an die Steuereinheit. Die Behandlung kann daraufhin mit dem nun aktiven Ultraschallkopf mit den vorgegebenen weiteren Parametern fortgesetzt werden.

Um die Kombination einer Beschallung des biologischen Gewebes mit Ultraschall mit einer Reizstrombehandlung zu ermöglichen, weist das System in einer weiteren vorteilhaften Ausführungsform einen Reizstromgenerator auf, welcher einen Reizstrom generiert und an einem Reizstromausgang ausgibt. Der Reizstromausgang ist mit einer Kontakteinheit, welche beispielsweise als an sich bekannte Elektrode ausgeführt sein kann, verbunden, sodass über die Kontakteinheit der Reizstrom auf das biologische Gewebe übertragen werden kann. Die Kontakteinheit ist in den Ultraschallkopf integriert, sodass durch Auflegen des Ultraschallkopfes auf das biologische Gewebe sowohl der Reizstrom als auch die Ultraschallstrahlung in das Gewebe eindringen können.

Ebenso ist eine Kombination des erfindungsgemäßen Systems zur Erzeugung von Ultraschallwellen mit einer Vakuumeinheit vorteilhaft. Hierzu weist das erfindungsgemäße System eine Vakuumeinheit mit einem Vakuumanschluss auf, wobei die Vakuumeinheit derart an dem Ultraschallkopf angeordnet ist, dass bei Anlegen des Ultraschallkopfes an das biologische Gewebe ein Unterdruck erzeugt werden kann, welcher das biologische Gewebe an den Ultraschallkopf andrückt. Die Vakuumeinheit kann beispielsweise als Vakuumglocke ausgebildet sein. Diese bildet mit dem Ultraschallkopf eine bauliche Einheit, so dass durch Auflegen des Ultraschallkopfes auf das biologische Gewebe automatisch eine annähernd fluiddichte Verbindung zwischen biologischem Gewebe und Vakuumglocke hergestellt werden kann.

Ebenso ist die Kombination des erfindungsgemäßen Systems zur Erzeugung von Ultraschallwellen mit einer Infraroteinheit zur Wärmebehandlung des biologischen Gewebes vorteilhaft. Hierzu weist das erfindungsgemäße System eine Infraroteinheit zur Erzeugung von Infrarotstrahlung auf, welche eine Infrarotstrahlungsquelle, wie beispielsweise eine Glühröhre, und eine Energieversorgung für Infrarotstrahlungsquelle umfasst. Die Infrarotstrahlungsquelle ist in den Ultraschallkopf integriert und derart ausgerichtet, dass bei Auflegen des Ultraschallkopfs auf das biologische Gewebe die Infrarotstrahlung der Infrarotstrahlungsquelle auf die Oberfläche des biologischen Gewebes einwirkt.

Bei dem erfindungsgemäßen Verfahren zur Erzeugung von Ultraschallwellen wird wie oben beschrieben ein periodisches elektrisches Signal mittels eines Signalgenerators erzeugt, welches mit Hilfe eines Ultraschallkopfes in Ultraschallwellen umgewandelt wird. Wesentlich ist, dass das erzeugte periodische elektrische Signal zwischen mindestens zwei Frequenzen umgeschaltet wird, wobei die Umschaltung automatisch erfolgt, insbesondere ohne das hierzu eine Eingabe des Benutzers notwendig wäre.

Besonders vorteilhaft ist es, wenn die periodischen elektrischen Signale im Bereich 20 kHz bis 20 MHz liegen, insbesondere im Bereich 0,5 MHz bis 10 MHz.

Die Umschaltfrequenz liegt vorteilhafterweise im Bereich 10 Hz bis 10 kHz, insbesondere im Bereich 100 Hz bis 2000 Hz.

Sofern zwischen 2 Ultraschallfrequenzen hin- und hergeschaltet wird, haben sich die Ultraschallfrequenzen 1 MHz und 3 MHz bzw. 3 MHz und 10 MHz als vorteilhaft erwiesen.

Ein Ausführungsbeispiel der Erfindung wird im Folgenden anhand der Figuren erläutert. Hierbei zeigen
- Figur 1: eine perspektivische Ansicht eines erfindungsgemäßen Ultraschallkopfes und
- Figur 2: ein Blockschaltbild eines erfindungsgemäßen Systems zur Erzeugung von Ultraschallwellen.

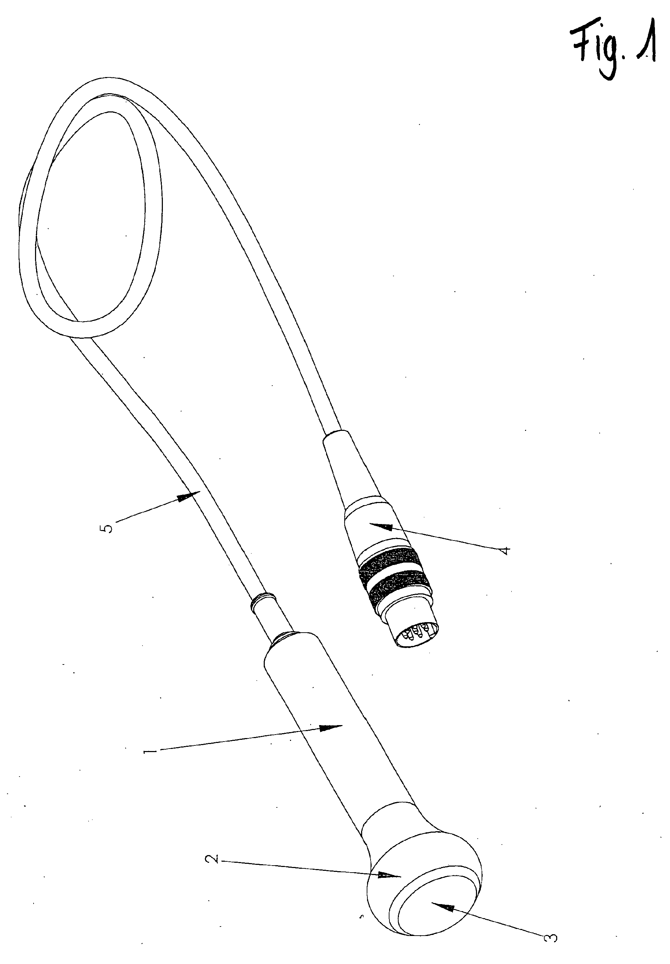

Der in Figur 1 dargestellte erfindungsgemäße Ultraschallkopf umfasst ein Gehäuse 1, welches an dem vorderen Ende ein Kopfteil 2 aufweist, an dem ein Protektor 3 angeordnet ist. An dem Protektor 3 ist eine Auflagefläche ausgebildet, zum Anlegen an das biologische Gewebe. Auf der im Kopfteil 2 liegenden Seite des Protektors 3 ist an dem Protektor ein Piezoelement angebracht, welches die Ultraschallschwingungen erzeugt und an den Protektor 3 weitergibt. In dem Gehäuse 1 ist ferner eine entsprechende Ansteuerelektronik für das Piezoelement enthalten, welche über eine Anschlussleitung 5 und einen Stecker 4 mit einem (nicht dargestellten) Signalgenerator verbunden werden kann. Die in dem Gehäuse 1 enthaltene Ansteuerelektronik wandelt in Verbindung mit dem Piezoelement elektrische Signale, welche über das Kabel 5 übertragen werden, in Ultraschallwellen um, sodass durch Auflegen des Protektors 3 auf biologisches Gewebe dieses mit Ultraschallwellen beaufschlagt werden kann.

Das Gehäuse 1 umfasst ferner eine Kopplungskontrolle 2. Die Kopplungskontrolle registriert, ob der Protektor 3 mit Druck beaufschlagt ist, das heißt ob der Protektor 3 an biologischem Gewebe anliegt. Ist dies der Fall, so gibt die Kopplungskontrolle 2 ein entsprechendes Signal über das Kabel 5 und den Stecker 4 an den (nicht dargestellten) Mikrocontroller des erfindungsgemäßen Systems zur Erzeugung von Ultraschallstrahlung.

In dem Stecker 4 befindet sich ferner ein Speicherbaustein, auf dem Kalibrierungsdaten des Ultraschallkopfes, insbesondere des Piezoelementes gespeichert sind. Der Speicherbaustein kann über in dem Stecker 4 angebrachte Kontakte mit dem Mikrocontroller verbunden werden, sodass der Mikrocontroller die gespeicherten Daten auslesen und abhängig von den Kalibrierungsdaten die Intensität der am Signalausgang des Signalgenerators anliegenden Signale derart wählen, dass eine Ultraschallstrahlung mit einer vorgegebenen Intensität durch das Piezoelement erzeugt wird.

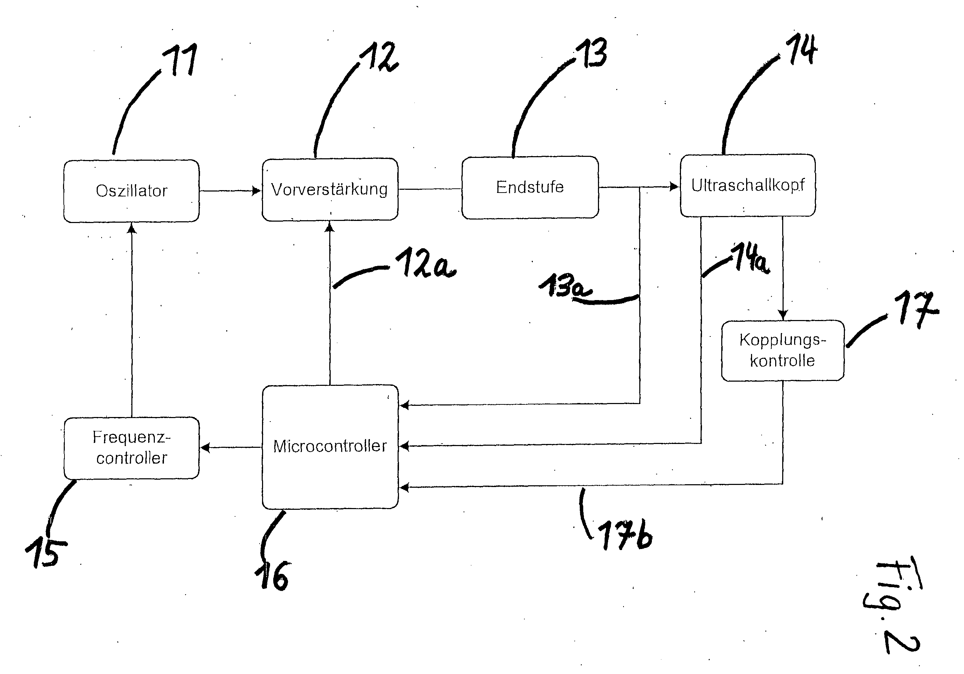

In Figur 2 ist ein Blockschaltbild des erfindungsgemäßen Systems zur Erzeugung von Ultraschallstrahlung dargestellt. Ein Oszillator 11 erzeugt periodische elektrische Signale, die anschließend in einer Verstärkungseinheit verstärkt werden. Diese Verstärkungseinheit besteht aus einer Vorverstärkung 12 und einer Endstufe 13. Das von der Endstufe ausgegebene Signal wird durch den in Figur 1 dargestellten Stecker 4 und das Kabel 5 an den Ultraschallkopf weitergeleitet, so dass im Ultraschallkopf 14 das elektrische Signal in Ultraschallwellen umgewandelt werden kann.

Die Frequenz, mit der der Oszillator 11 schwingt, wird durch einen Frequenzcontroller 15 reguliert. Dieser wird wiederum von einer Steuereinheit 16, welche als Microcontroller ausgeführt ist gesteuert, welcher die aktuell zu erzeugende Frequenz vorgibt. Für den Betrieb sind in dem Mikrocontroller folgende Größen vorgegeben:
- Die gewünschte Intensität der Ultraschallstrahlung,
- die Ultraschallfrequenzen, zwischen denen gewechselt werden soll,
- die Reihenfolge, in der zwischen den vorgegebenen Ultraschallfrequenzen gewechselt werden soll und
- die Umschaltfrequenz, mit der die Ultraschallfrequenzen gemäß der vorgegebenen Reihenfolge gewechselt werden.

Sind beispielsweise drei Ultraschallfrequenzen 1 MHz, 2 MHz und 3 MHz, eine Wechselreihenfolge (1 MHz nach 2 MHz, 2 MHz nach 3 MHz, 3 MHz nach 1 MHz), eine Intensität 1 W/cm², und eine Wechselfrequenz von 100 Hz vorgegeben, so gibt der Mikrocontroller zunächst für 0,01 sec das Signal an den Frequenzcontroller 15 zur Erzeugung einer Frequenz mit 1 MHz, danach für 0,01 sec das Signal zur Erzeugung einer Frequenz mit 2 MHz, dann für 0,01 sec das Signal zur Erzeugung einer Frequenz mit 3 MHz und wiederholt diesen Zyklus fortlaufend.

Demgemäß wird ein entsprechendes periodisches Signal mit der gewünschten Frequenz erzeugt und liegt am Ausgang der Endstufe 13 an. Dieser Ausgang ist über eine Leitung 13a mit einem Signaleingang des Mikrocontrollers verbunden, sodass im Mikrocontroller die tatsächlich an der Endstufe anliegende Signalstärke bestimmt werden kann. Der Mikrocontroller gibt nun über die Leitung 12a ein entsprechendes Signal an die Vorverstärkung 12, sodass am Ausgang der Endstufe 13 ein Signal mit der gewünschten Intensität anliegt. Zur Berechnung der gewünschten Intensität des am Ausgang der Endstufe 13 anliegenden elektrischen Signals werden von dem Mikrocontroller über die Leitung 14a die Kalibrierungsdaten des Ultraschallkopfes eingelesen. Mit Hilfe dieser Kalibrierungsdaten berechnet der Mikrocontroller die gewünschte Intensität am Ausgang der Endstufe 13 derart, dass durch den Ultraschallkopf Ultraschallstrahlung mit der vorgegebenen Intensität von 1 W/cm² erzeugt wird.

Darüber hinaus ist der Mikrocontroller über eine Leitung 17b mit der Kopplungskontrolle 7 des Ultraschallkopfes 4 verbunden. Die Kopplungskontrolle gibt bei Anliegen des in Figur 1 dargestellten Protektors 3 an biologischem Gewebe ein Signal an den Mikrocontroller und der Mikrocontroller ist derart ausgeführt, dass er nur bei anliegendem Signal der Kopplungskontrolle ein positives Signal an die Vorverstärkung abgibt. Dies bedeutet, dass bei fehlendem Signal der Kopplungskontrolle, das heißt, wenn der in Figur 1 dargestellte Protektor 3 nicht an dem biologischen Gewebe anliegt, kein positives Signal an die Vorverstärkung abgegeben wird und somit keinerlei Verstärkung stattfindet, sodass am Ausgang der Endstufe 3 allenfalls ein Signal mit geringer Intensität anliegt.

## Patentansprüche

1. System zur Erzeugung von Ultraschallwellen, insbesondere zur kosmetischen und therapeutischen Behandlung mit einem elektrisch angesteuerten Ultraschallkopf (14), der Ultraschallwellen mit zumindest zwei unterschiedlichen Frequenzen *f₁, f₂* erzeugt,
**dadurch gekennzeichnet,**
**dass** das System eine Steuereinheit (16) aufweist, die die mindestens zwei Frequenzen der Ultraschallwellen permanent oder zumindest während vorgegebener Zeitspannen zwischen den zumindest zwei vorgegebenen Frequenzwerten *f₁, f₂* umschaltet.

2. System zur Erzeugung von Ultraschallwellen nach Anspruch 1,
**dadurch gekennzeichnet,**
**dass** es einen Signalgenerator (11, 12, 13, 15) aufweist, der zumindest zwei Frequenzen *f₁*, *f₂* erzeugt, die in Ultraschallwellen mit entsprechenden Frequenzen umgewandelt werden und dass die Steuereinheit (16) die Umschaltung zwischen den Frequenzen bewirkt.

3. System zur Erzeugung von Ultraschallwellen nach Anspruch 2,
**dadurch gekennzeichnet,**
**dass** der Signalgenerator einen Oszillator und eine Frequenzmodulations-Einheit umfasst, die aus der Oszillator-Frequenz zumindest zwei verschiedene Frequenzen erzeugt.

4. System zur Erzeugung von Ultraschallwellen nach einem der vorangegangenen Ansprüche,
**dadurch gekennzeichnet,**
**dass** die Steuereinheit (16) derart ausgeführt ist, dass die Ultraschallfrequenz mit einer Wechselfrequenz im Bereich von 10 Hz bis 10 kHz, insbesondere im Bereich 100 Hz bis 2000 Hz zwischen den mindestens zwei Werten *f₁* und *f₂* wechselt.

5. System zur Erzeugung von Ultraschallwellen nach einem der vorangegangenen Ansprüche,
**dadurch gekennzeichnet,**
**dass** der Signalgenerator einen Oszillator (11), einen Frequenzcontroller (15) und eine Verstärkungseinheit (12, 13) umfasst,
wobei der Frequenzcontroller (15) mit dem Oszillator (11) verbunden und derart ausgeführt ist, dass er für eine vorgegebene Frequenz derart auf den Oszillator (11) einwirkt, dass dieser mit der vorgegebenen Frequenz schwingt und
wobei ein Ausgang des Oszillators (11) mit einem Eingang der Verstärkungseinheit (12, 13) verbunden und die Verstärkungseinheit derart ausgeführt ist, dass sie das am Eingang anliegende Signal in etwa um einen vorgegebnen Faktor verstärkt und
**dass** ein Ausgang der Verstärkungseinheit (12, 13) der Signalausgang des Signalgenerators ist.

6. System zur Erzeugung von Ultraschallwellen nach Anspruch 5,
**dadurch gekennzeichnet,**
**dass** die Steuereinheit (16) als Microcontroller mit einem Steuerausgang ausgeführt ist, welcher mit einem Eingang des Frequenzcontrollers (15) verbunden ist, zur Steuerung der durch den Signalgenerator erzeugten Frequenz.

7. System zur Erzeugung von Ultraschallwellen nach Anspruch 6,
**dadurch gekennzeichnet,**
**dass** die Steuereinheit (16) mit einem Steuereingang der Verstärkungseinheit (12, 13) verbunden ist,
**dass** die Steuereinheit (16) einen Signaleingang aufweist, welcher mit dem Signalausgang der Verstärkungseinheit (12, 13) verbunden ist und dass die Steuereinheit (16) derart ausgeführt ist, dass sie abhängig von der Signalstärke des am Signaleingang der Steuereinheit (16) anliegenden Signals derart über den Steuereingang der Verstärkungseinheit (12, 13) auf diese einwirkt, dass die Verstärkungseinheit (12, 13) ein periodisches elektrisches Signal mit einer durch die Steuereinheit (16) vorgegebenen Intensität erzeugt.

8. System zur Erzeugung von Ultraschallwellen nach einem der vorangegangenen Ansprüche,
**dadurch gekennzeichnet,**
**dass** das System mindestens zwei Ultraschallköpfe (14) insbesondere mit unterschiedlicher Größe aufweist und
**dass** der Signalausgang des Signalgenerators derart ausgeführt ist, dass wahlweise ein oder die mindestens zwei Ultraschallköpfe (14) gleichzeitig mit dem Signalausgang des Signalgenerators verbunden werden können.

9. System zur Erzeugung von Ultraschallwellen nach Anspruch einem der vorangegangenen Ansprüche,
**dadurch gekennzeichnet,**
**dass** das System zusätzlich einen Reizstromgenerator aufweist, mit einem Reizstromausgang und
**dass** der Ultraschallkopf (14) eine Kontakteinheit zur Übertragung eines Reizstromes auf biologisches Gewebe aufweist und derart ausgeführt ist, dass die Kontakteinheit an den Reizstromausgang des Reizstromgenerators anschließbar ist.

10. System zur Erzeugung von Ultraschallwellen nach einem der vorangegangenen Ansprüche,
**dadurch gekennzeichnet,**
**dass** das System zusätzlich eine Vakuumeinheit aufweist mit einem Vakuumanschluss zum Erzeugen eines Unterdrucks und
**dass** der Ultraschallkopf (14) eine Vakuumglocke umfasst und derart ausgeführt ist, dass die Vakuumglocke an den Vakuumanschluss der Vakuumeinheit anschließbar ist, wobei der Ultraschallkopf und die Vakuumglocke derart ausgeführt sind, dass bei Anlegen des Ultraschallkopfes an biologisches Gewebe die Vakuumglocke nahezu fluiddicht an dem biologischen Gewebe anliegt, so dass zwischen biologischem Gewebe und Ultraschallkopf ein Unterdruck hergestellt werden kann.

11. System zur Erzeugung von Ultraschallwellen nach einem der vorangegangenen Ansprüche,
**dadurch gekennzeichnet,**
**dass** das System zusätzlich eine Infraroteinheit zur Erzeugung von Infrarotstrahlung aufweist, umfassend mindestens eine Infrarotstrahlungsquelle zur Erzeugung der Infrarotstrahlung und eine mit der Infrarotstrahlungsquelle verbundene Energieversorgung,
wobei die Infrarotstrahlungsquelle mit dem Ultraschallkopf (14) eine bauliche Einheit bildet.

12. System zur Erzeugung von Ultraschallwellen nach einem der vorangegangenen Ansprüche,
**dadurch gekennzeichnet,**
**dass** der Signalgenerator derart ausgeführt ist, dass er periodische elektrische Signale mit Frequenzen im Bereich 20 kHz bis 20 MHz, insbesondere in den Bereichen 20 kHz bis 100 kHz und 0,7 MHz bis 10 MHz erzeugt.

13. Verfahren zur Erzeugung von Ultraschallwellen insbesondere zur kosmetischen Behandlung, folgende Schritte umfassend:
A Erzeugen eines periodischen elektrischen Signals mittels eines Signalgenerators,
B Umwandeln des in Schritt A erzeugten elektrischen Signals in Ultraschallwellen mittels eines Ultraschallkopfes (14),
**dadurch gekennzeichnet,**
**dass** in Schritt A abwechselnd periodische elektrische Signale mit mindestens zwei unterschiedlichen Frequenzen *f₁* und *f₂* erzeugt werden,
wobei zwischen den mindestens zwei Frequenzen automatisch und permanent oder zumindest während vorgegebener Zeitspannen umgeschaltet wird.

14. Verfahren zur Erzeugung von Ultraschallwellen nach Anspruch 13,
**dadurch gekennzeichnet,**
**dass** die Frequenzen der periodischen elektrischen Signale im Bereich 20 kHz bis 20 MHz liegen, insbesondere im Bereich 0.5 MHz bis 10 MHz.

15. Verfahren zur Erzeugung von Ultraschallwellen nach einem der Ansprüche 13 bis 14,
**dadurch gekennzeichnet,**
**dass** zwischen den mindestens zwei Frequenzen mit einer Umschaltfrequenz im Bereich von 10 Hz bis 10 kHz, insbesondere im Bereich 100 Hz bis 2000 Hz umgeschaltet wird.

16. Verfahren zur Erzeugung von Ultraschallwellen nach einem der Ansprüche 13 bis 15,
**dadurch gekennzeichnet,**
**dass** die Frequenz *f₁* in etwa 1 MHz und die Frequenz *f₂* in etwa 3 MHz ist oder
**dass** die Frequenz *f₁* in etwa 3 MHz und die Frequenz *f₂* in etwa 10 MHz ist.
